# EUROPEAN PATENT APPLICATION

(11) **EP 1 570 802 A2**
(43) Date of publication of application: **07.09.2005**
(21) Application number: 05004837.0
(22) Date of filing: 04.03.2005
(51) Int. Cl.: A61B 19/00

(54) **Adjustable navigated tracking element mount**

(30) Priority: 05.03.2004 US 550460 P; 09.09.2004 US 937166
(71) Applicant: Zimmer Technology, Inc., Illinois 60606 (US)
(72) Inventor: Grimm, James E., Winona Lake, IN 46590 (US); Hui, Sudip, Warsaw, IN 46582 (US); Rangaiah, Chetan, Warsaw, IN 46582 (US); Hall, Maleata Y., Warsaw, IN 46580 (US); McGinley, Shawn E., Fort Wayne, IN 46814 (US); Walriven, Dale E., Warsaw, IN 46850 (US)
(74) Representative: Manitz, Finsterwald & Partner GbR

(57) **Abstract**

An adjustable navigated tracking element mount and method are provided.

## Description

### BACKGROUND

The present invention relates to surgical components used in conjunction with a surgical navigation system. In particular, the present invention relates to an adjustable navigated tracking element mount.

Many surgical procedures are now performed with surgical navigation systems in which sensors detect tracking elements attached in known relationship to a surgical component in the surgical suite such as a surgical instrument, implant, or patient body part. The sensor information is fed to a computer that then triangulates the three dimensional position of the tracking elements within the surgical navigation system coordinate system. Thus, the computer can resolve the position of the surgical component and display the position for surgeon guidance. For example, the position can be shown superimposed on an image of the patient's anatomy obtained via X-ray, CT scan, ultrasound, or other imaging technology. Likewise, positional data may be provided in the form of textual or numerical readouts for surgeon reference.

The mounting position of the tracking elements on the surgical component to be tracked may affect the ability of the surgical navigation system to detect the tracking elements. Similarly, there may be a mounting position that lessens interference with other equipment in the surgical suite and/or with anatomical structures at the surgical site. Also, the mounting position may be a matter of convenience and/or comfort for the surgical personnel.

### SUMMARY

The present invention provides an adjustable navigated tracking element mount.

In one aspect of the invention, an adjustable mount is provided for attaching one or more tracking elements to a surgical component to be tracked by a surgical navigation system during a surgical procedure. The adjustable mount includes a first member including means for attaching the first member to the surgical component to be tracked. The mount further includes a second member including means for attaching the second member to the one or more tracking elements. The second member is connected to the first member for relative motion between the first and second members. The first and second members are relatively moveable between a plurality of relative positions. The mount further includes means for releasably locking the first and second members in one of the plurality of relative positions.

In another aspect of the invention, a combination is provided for use with a surgical navigation system during a surgical procedure. The combination includes a surgical component, an array of tracking elements trackable by the surgical navigation system, and an adjustable mount for attaching the array to the surgical component. The mount includes a first member connected to the surgical component and a second member connected to the array. The first and second members are connected for relative motion between the first and second members to permit repositioning of the array relative to the surgical component. The first and second members are relatively moveable between a plurality of relative positions.

The invention can be used in a connection with a method of performing a surgical procedure at a surgical site of a patient's body which includes: providing an adjustable mount having first and second members, one of the members being attached to a surgical component to be tracked by a surgical navigation system and the other of the members being attached to at least one tracking element, the first and second members being mounted for relative motion between a plurality of relative positions; adjusting the relative position of the first and second members to place the at least one tracking element in a desired position relative to the surgical component; and tracking the at least one tracking element with a surgical navigation system.

Preferably, the method further comprises: adjusting the relative position of the first and second members intraoperatively to change the position of the at least one tracking element to accommodate changed surgical circumstances.

Preferably, the first and second members include opposed teeth and corresponding notches biased into engagement to lock the relative position of the first and second members, the step of adjusting comprising: separating the teeth and notches; adjusting the relative position of the first and second members; and releasing the first and second members such that the teeth and notches are biased together to lock the new position.

Preferably, the at least one tracking element comprises an element detected by imaging and adjusting the relative position of the first and second members places the at least one tracking element in the line of sight of an imaging sensor.

### BRIEF DESCRIPTION OF THE DRAWINGS

Various embodiments of the present invention will be discussed with reference to the appended drawings. These drawings depict only illustrative embodiments of the invention and are not to be considered limiting of its scope.

FIG. 1 is a side elevation view of an illustrative adjustable navigated tracking element mount according to the present invention;

FIG. 2 is a bottom plan view of the adjustable navigated tracking element mount of FIG. 1; and

FIG. 3 is a side elevation view of the adjustable navigated tracking element mount of FIG. 1 shown connecting an array of tracking elements to a surgical instrument.

### DESCRIPTION OF THE ILLUSTRATED EMBODIMENTS

Embodiments of an adjustable navigated tracking element mount may be configured to provide a plurality of mounting positions for one or more tracking elements for use with a surgical navigation system. The mount may include moveable members that are adjustable to change the position of the tracking elements relative to a surgical component on which they are mounted. For example, the mount may include a first member connectable to the tracked surgical component and a second member connectable to the tracking elements. The second member may be connectable to the first member in a plurality of positions. For example, a pivoting, rotating, sliding, and/or other joint may be incorporated into the mount. The movable members may be relatively infinitely adjustable or may be adjustable to a finite number of discrete positions. For example, a rotating joint may include intermeshing teeth and notches that lock the joint in discrete angles of rotation. The mount may include additional members mounted between the tracking elements and tracked surgical component to permit additional degrees of adjustment. For example, a third member may provide additional translational and/or rotational adjustment of the relative position of the tracking elements and tracked surgical component.

The mount may include an attachment mechanism to connect one or more tracking elements to the mount. For example, an attachment screw, bolt, boss, pin, dovetail, bayonet mount, magnet, weld, adhesive, hook and loop material, and/or other suitable attachment mechanism may be included. For example, an array of tracking elements may include a mounting block having a female dovetail and the mount may include a male dovetail engageable with the female dovetail.

The mount may include an attachment mechanism to connect the mount to the tracked surgical component. For example, an attachment screw, bolt, boss, pin, dovetail, bayonet mount, magnet, weld, adhesive, hook and loop material, and/or other suitable attachment mechanism may be included. For example, a boss may extend from the mount to engage a hole in a surgical instrument. In addition, an antirotation pin, offset from the boss, may extend from the mount to engage a second hole in the surgical instrument so prevent the mount from rotating. A screw may engage the mount and the surgical instrument to tighten the assembly.

The mount may be connected to the tracking elements and the tracked surgical component and calibrated prior to surgery. The mount may be adjusted to reposition the tracking elements during surgery to accommodate changing surgical circumstances such as the position of the patient, surgical equipment, surgical personnel and/or to accommodate other needs. The new relationship between the tracking elements and the tracked surgical component may be calibrated by placing the surgical component in contact with a calibration device or otherwise in a position known to the surgical navigation system. The mount may also be configured with predefined discrete positions that are entered into the surgical navigation system prior to surgery. When the mount is changed intraoperatively to a new position, the new position may be automatically detected or selected from a menu in the surgical navigation system.

The mount may be used with one or more tracking elements. The elements may be mounted in an array on a tracking element array base. The tracking elements may be detectable electromagnetically, acoustically, by imaging, and/or by other suitable detection means. Furthermore, the tracking element may be active or passive. Examples of active tracking elements may include electromagnetic elements in an electromagnetic system, light emitting diodes in an imaging system, and ultrasonic emitters in an acoustic system, among others. Examples of passive tracking elements may include elements with reflective surfaces. For example, reflective spheres or discs may be attached to the orthopaedic guide and detected by an imaging system.

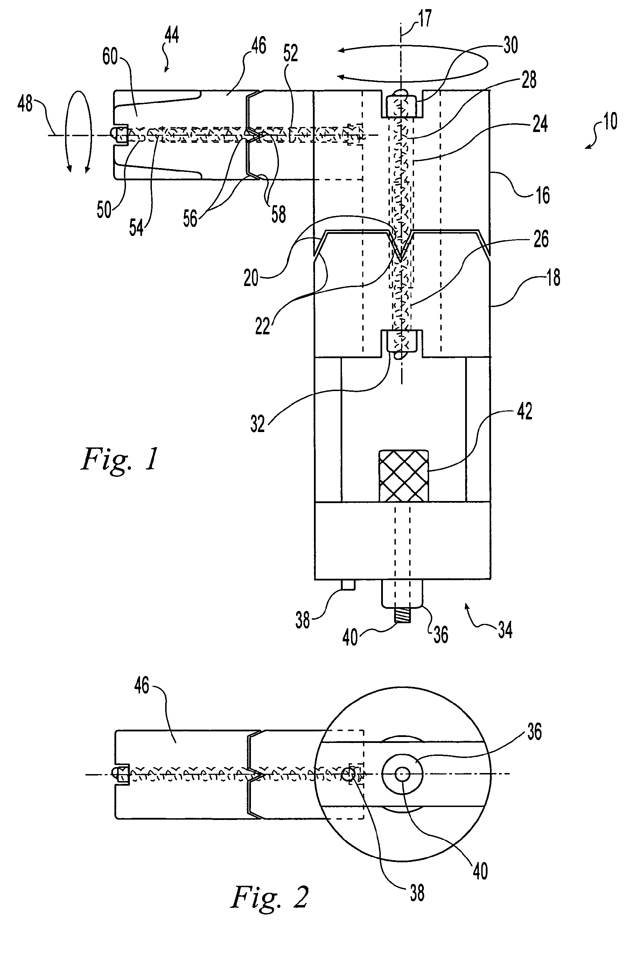

FIGS. 1-3 depict an illustrative adjustable navigated tracking element mount 10 for attaching one or more tracking elements 12 to a surgical component to be tracked. In the illustrative example, the mount 10 is shown attaching an array 14 of image guided tracking elements 12 to an acetabular cup inserter 100. The mount 10 of the present invention may be used to attach any number or kind of tracking elements to any surgical component to be tracked.

The illustrative mount 10 includes a first upper member 16 and a second lower member 18 mounted to one another for relative rotation about an axis 17. Circumferentially arranged teeth 20 and corresponding grooves 22 position the upper and lower members 16, 18 in discrete rotated positions. First and second telescoping tubes 24, 26 are biased inwardly by a coil spring 28. The tubes have heads 30, 32 that abut the upper and lower members 16, 18 to bias them into axial engagement with the teeth 20 seated in the grooves 22. The upper and lower members 16, 18 may be repositioned by biasing them apart axially against the spring 28 tension to disengage the teeth 20 from the grooves 22. The upper and lower members 16, 18 may then be rotated to a new rotational position and released such that the spring tension reengages the teeth 20 and grooves 22. Alternatively, the upper and lower members 16, 18 may maintain their rotational positions by means of other positive engagement features and/or friction. Likewise, the upper and lower members may include an axial locking bolt or other axial compression mechanism in place of, or in combination with, the spring 28 and tubes 24, 26.

The lower member 18 includes an attachment mechanism 34 for connecting the mount to a surgical component to be tracked and lock all six possible degrees of freedom between the mount and surgical component. The illustrative attachment mechanism includes a projecting boss 36 for engaging a corresponding hole in the surgical component to control four degrees of freedom including translation along two axes and rotation about two axes.
An antirotation pin 38 is offset from the boss 36 and projects from the attachment mechanism 34 to engage a corresponding hole in the surgical component to control the third rotational degree of freedom. A locking screw 40 threads into the surgical component to control the third translational degree of freedom and lock the mount 10 onto the surgical component. The screw 40 includes a knob 42 that abuts the lower member 18 of the mount to clamp it onto the surgical component.

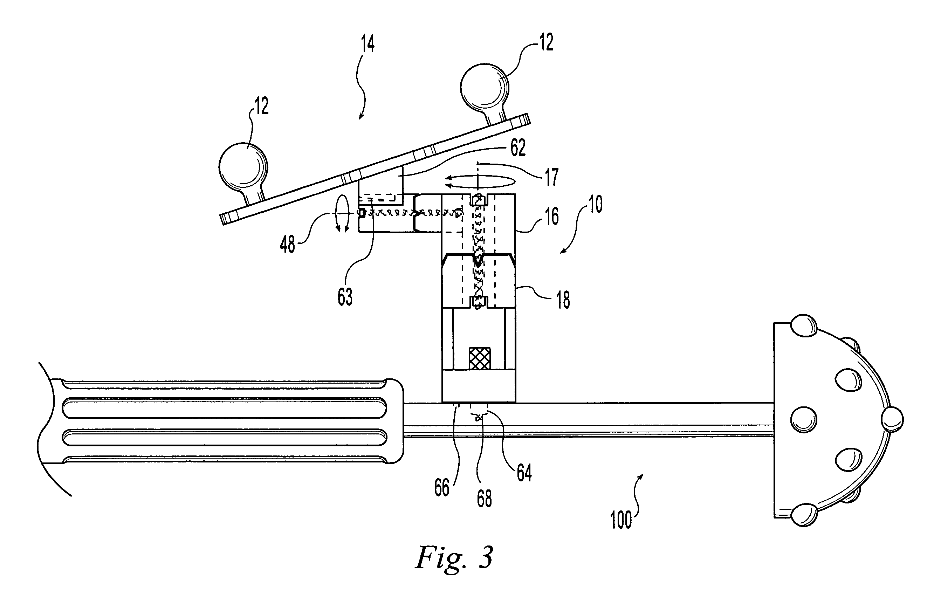

The upper member 16 of the mount 10 connects to one or more tracking elements 12 (FIG. 3) to thereby mount the tracking elements 12 onto the tracked surgical component.
The illustrative tracking element attachment mechanism 44 includes a male dovetail member 60 for matingly engaging a female dovetail member attached to the tracking elements 12.
The gender of the mechanism may be reversed and other mechanisms may be used to connect the components. The position of the tracking elements 12 relative to the tracked surgical component may be adjusted by repositioning the upper and lower members 16, 18 of the mount relative to one another. In the illustrative mount 10, a tracking element attachment mechanism 44 is connected to the upper member 16 by means of a tracking element rotation member 46 mounted on the upper member 16 for rotation about an axis 48. The rotational position of the tracking element rotation member 46 relative to the upper member 16 may be controlled using a similar mechanism as that between the upper and lower members 16, 18.
A pair of telescoping tubes 50, 52 compressed inwardly by a spring 54 acts to bias circumferential teeth 56 into corresponding notches 58. The rotational position of the tracking element rotation member 46 relative to the upper member 16 may be adjusted by pulling the tracking element rotation member axially away from the upper member 16 to disengage the teeth 56 and grooves 58. The tracking element rotation member 46 may then be rotated to a new position and released so that the teeth 56 and grooves 58 reengage. The rotational axis 48 of the tracking element rotation member 46 is transverse to the rotational axis 17 of the lower member so that by adjusting rotation about both axes 48, 17, the tracking element position 12 may be changed three dimensionally. The total number of possible positions for the tracking elements 12 is determined by the number of positions about each rotation axis. For example, if each rotation axis 48, 17 is configured for a total of four discrete rotational positions, then there would be sixteen possible positions for the tracking elements 12 relative to the lower member 18 and tracked surgical component.

FIG. 3 depicts the illustrative adjustable navigated tracking element mount 10 in use to connect an array 14 of tracking elements 12 to a surgical component in the form of an acetabular cup inserter 100. The array 14 includes a mounting block 62 having a dovetail 63 for mating with the dovetail 60 of the tracking element rotation member 46. The acetabular cup inserter 100 includes holes 64, 66, 68 for receiving the boss 36, antirotation pin 38, and locking screw 40.

In use, the array 14 is attached to the navigated tracking element mount 10 by engaging the dovetails 60, 63. The mount 10 is attached to the acetabular cup inserter 100 by engaging the boss 36 with its corresponding hole 64, the antirotation pin 38 with its corresponding hole 66, and the locking screw 40 with its corresponding hole 68. The knob 42 of the locking screw 40 is turned to tighten the attachment. The position of the array 14 may be adjusted by rotating the mount assembly about each of the axes 17, 48. For example, the array 14 position may be adjusted to facilitate the best signal reception by the surgical navigation system. For example, in an image guided system, the array 14 position may be adjusted so that the line of sight between the system cameras and the tracking elements 12 are unobstructed. The array 14 position may also be adjusted so that the array 14 does not impinge on adjacent anatomical structures, such as the flesh at the borders of a surgical wound, or other surgical equipment. The array 14 position may also be adjusted to accommodate a surgeons grip on a surgical component, such as the acetabular cup inserter 100. The array 14 position may be adjusted one or more times during surgery as the need arises. For example, the position may be adjusted to accommodate the need to reposition the patient, movement of the surgeon from one side of the body to another, movement of the surgeon between standing and sitting positions, moving surgical equipment, and/or to accommodate other needs. The new relationship between the array 14 and tracked surgical component may be calibrated by placing the surgical component in contact with a calibration device or in a position known to the surgical navigation system. The mount may be configured with predefined discrete positions that are entered into the surgical navigation system prior to surgery. When the mount is changed intraoperatively to a new position, the new position may be automatically detected or selected from a menu in the surgical navigation system.

Although examples of an adjustable navigated tracking element mount and its use have been described and illustrated in detail, it is to be understood that the same is intended by way of illustration and example only and is not to be taken by way of limitation. The invention has been illustrated in use to attach an array of tracking elements to an acetabular cup inserter. However, the adjustable navigated tracking element mount may be configured for use with any surgical component that one desires to track, at other surgical locations, to perform other functions. Accordingly, variations in and modifications to the adjustable navigated tracking element mount and its use will be apparent to those of ordinary skill in the art, and the following claims are intended to cover all such modifications and equivalents.

## Claims

1. An adjustable mount for attaching one or more tracking elements to a surgical component to be tracked by a surgical navigation system during a surgical procedure, the adjustable mount comprising:
a first member including means for attaching the first member to the surgical component to be tracked;
a second member including means for attaching the second member to the one or more tracking elements, the second member being connected to the first member for relative motion between the first and second members, the first and second members being relatively moveable between a plurality of relative positions; and
means for releasably locking the first and second members in one of the plurality of relative positions.

2. The adjustable mount of claim 0 wherein the first and second members are mounted for relative rotation about a first axis and the first and second members are rotatable between a finite number of discrete rotated positions.

3. The adjustable mount of claim 2 wherein one of the first and second members includes teeth and the other of the first and second members includes corresponding notches, the teeth and notches being engageable to position the first and second members in a finite number of positions.

4. The adjustable mount of claim 3 further including a spring member biasing the first and second members together to engage the teeth with the notches.

5. The adjustable mount of claim 0 wherein the means for attaching the second member to the one or more tracking elements includes a third member connected to the second member for independent motion relative to the second member; and means for releasably locking the relative position of the third member relative to the second member.

6. The adjustable mount of claim 5 wherein the second and third members are mounted for relative rotation about a second axis transverse to the first axis and the second and third members are rotatable between a finite number of discrete rotated positions.

7. A combination to be used with a surgical navigation system during a surgical procedure, the combination comprising:
a surgical component;
an array of tracking elements trackable by the surgical navigation system; and
an adjustable mount for attaching the array to the surgical component, the mount including a first member connected to the surgical component and a second member connected to the array, the first and second members being connected for relative motion between the first and second members to permit repositioning of the array relative to the surgical component, the first and second members being relatively moveable between a plurality of relative positions.

8. The combination of claim 7 wherein the first and second members are mounted for relative rotation about a first axis between a plurality of relative positions.

9. The combination of claim 8 wherein the first and second members are rotatable between a finite number of discrete relative positions, the combination further comprising a locking mechanism for releasably locking the first and second members in a desired one of the discrete relative positions.

10. The combination of claim 7 further including:
a third member connected between the second member and the array for independent motion relative to the second member to provide an additional degree of adjustment of the array relative to the surgical component.

11. The combination of claim 10 wherein the first member is connected to the second member for relative rotation about a first axis and the second member is connected to the third member for relative rotation about a second axis transverse to the first axis.

12. The combination of claim 10 wherein the third member includes a dovetail connecting member and the array includes a corresponding dovetail connecting member, the dovetail connecting members being in engagement with one another.

13. The combination of claim 7 wherein the surgical component includes first, second, and third holes and the first member further comprises:
a boss extending from the first member, the boss engaging the first hole in the surgical component;
a pin extending from the first member, the pin being spaced from the boss, the pin engaging the second hole in the surgical component; and
a locking screw extending from the first member, the locking screw threadably engaging the third hole in the surgical component.

14. The combination of claim 7 wherein the array of tracking elements comprises tracking elements detectable by an image sensor.
